# EUROPÄISCHE PATENTANMELDUNG

(11) **EP 4 802 983 A1**
(43) Veröffentlichungstag der Anmeldung: **09.09.2026**
(21) Anmeldenummer: 25161622.3
(22) Anmeldetag: 04.03.2025
(51) Int. Cl.: A61B 1/00, A61B 1/018, A61B 17/00

(54) **STEUEREINHEIT FÜR EIN ENDOSKOP SOWIE ZUBEHÖR UND ENDOSKOPSYSTEM**

(71) Anmelder: Technische Universität Dresden Körperschaft des öffentlichen Rechts, 01069 Dresden (DE); Leibniz-Institut für Polymerforschung Dresden e.V., 01069 Dresden (DE); Wolfram Designer und Ingenieure, 01139 Dresden (DE)
(72) Erfinder: Hampe, Jochen, 01277 Dresden (DE); Brinkmann, Franz, 01309 Dresden (DE); Göbel, Steffen, 01099 Dresden (DE); Richter, Andreas, 01219 Dresden (DE); Körbitz, René, 01099 Dresden (DE); Henkel, Konrad, 01187 Dresden (DE); Pietsch, Malte Sören, 01099 Dresden (DE); Bruk, Sascha, 01099 Dresden (DE); Brückner, Stefan, 01279 Dresden (DE); Uhlig, Kai, 01109 Dresden (DE); Babatz, Jana, 01309 Dresden (DE); Wolfram, Sebastian, 01157 Dresden (DE); Hüttner, Ronny Andreas, 01099 Dresden (DE)
(74) Vertreter: Witte, Weller und Partner Patentanwälte mbB Stuttgart

(57) **Zusammenfassung**

Zur Verbesserung der manuellen Steuerung eines transendoskopischen Instruments wird eine neue Form einer Steuereinheit (10) vorgeschlagen.

Diese Steuereinheit (10) weist mindestens ein Steuerelement (20) auf, insbesondere in Form mindestens eines 2-Wege-Controllers (20) oder 4-Wege-Controllers (20) und/oder mindestens eines Betätigungsknopfes.

Die Steuereinheit (10) ist zur Anbringung an der Außenseite eines Endoskopschlauchs (110) oder zumindest zur dortigen Anlage ausgebildet und verfügt über einen Aufnahmebereich (14) oder einen Anlagebereich (16) für den Endoskopschlauch (110).

Vorgeschlagen werden insbesondere auch verschiedene mit dem Endoskopschlauch (110) zusammenwirkende Befestigungsmechanismen (50).

## Beschreibung

### ANWENDUNGSGEBIET UND STAND DER TECHNIK

Die Erfindung betrifft den Bereich der medizinischen Endoskope. Medizinische Endoskope sind Geräte, die zur visuellen Untersuchung und zur Behandlung im Körper eines Patienten eingesetzt werden. Sie weisen üblicherweise einen flexiblen Endoskopschlauch mit einer Lichtquelle und einer Kamera oder einem anderweitigen optischen System an der Endoskopspitze auf, die Bilder aus dem Körperinneren auf einen Monitor übertragen.

Medizinische Endoskope dienen üblicherweise auch der unmittelbaren Behandlung. Hierfür weisen Endoskope einen Arbeitskanal auf, der sich innerhalb des Endoskopschlauchs bis zur Endoskopspitze erstreckt. Durch diesen Arbeitskanal wird ein transendoskopisches Instrument bis zum distalen Ende geschoben und ragt dort aus der Endoskopspitze hervor. Solche Instrumente können als verschiedene Werkzeuge beschaffen sein, beispielsweise als endoskopische Zangen oder als Biopsiezangen.

Bei der Nutzung des Endoskops führt der Operateur das Endoskop üblicherweise derart, dass er mit der linken Hand den Steuerkopf des Endoskops hält, so dass er mit den Fingern der linken Hand die dortigen Bedienelemente zur Steuerung der Endoskopspitze handhaben kann. Der Operateur steuert hierüber insbesondere die Ausrichtung der Endoskopspitze.

Mit der anderen Hand, üblicherweise der rechten Hand, führt der Operateur den Endoskopschlauch selbst, wobei dies insbesondere ein Einschieben und Herausziehen des Endoskopschlauchs aus dem Körper sowie eine Torsion des Endoskopschlauchs um seine Längsachse betrifft.

Somit sind meist beide Hände des Operateurs für die Führung und Steuerung des Endoskops erforderlich. Gleichzeitig ist es jedoch häufig erforderlich, das in den Arbeitskanal des Endoskops eingeführte Instrument gezielt zu steuern bzw. zu betätigen. Da beide Hände des Operateurs bereits für die Führung des Endoskops gebunden sind, steht dem Operateur keine weitere Hand zur Steuerung des Instruments zur Verfügung. Dies stellt eine erhebliche Einschränkung dar, da für viele medizinische Eingriffe eine präzise und simultane Handhabung sowohl des Endoskops selbst als auch des eingesetzten Instruments erforderlich ist. Üblicherweise wird dieses Problem durch eine Assistenzperson gelöst, die das Instrument bedient. Dies erzwingt jedoch einen dauernden Austausch zwischen dem Operateur und dem Assistenten, was die Operation erschwert und Missverständnisse mit sich bringen kann.

### AUFGABE UND LÖSUNG

Aufgabe der Erfindung ist es daher, technische Mittel bereitzustellen, um endoskopische Operationen zu vereinfachen.

Zu diesem Zweck wird gemäß der vorliegenden Erfindung primär eine Steuereinheit zur manuellen Steuerung des transendoskopischen Instruments vorgeschlagen.

Diese Steuereinheit ist zur Steuerung eines transendoskopischen Instruments vorgesehen, welches durch den Arbeitskanal eines Endoskops in den Körper eines Patienten ragt.

Die Steuereinheit dient der Bedienung des Instruments. Sie weist hierfür mindestens ein Steuerelement auf. Was für ein Steuerelement dies ist, hängt primär von der Art des transendoskopischen Instruments ab. Bei sehr einfachen Instrumenten, die durch isolierte Steuerimpulse gesteuert werden, beispielsweise Zangen, die über einen solchen Impuls eine Greifbewegung vollziehen, kann im Einzelfall ein einzelner digital schaltender Knopf als Steuerelement ausreichen.

Zumeist sind jedoch vielfältigere Steuermöglichkeiten erforderlich, beispielsweise Steuermöglichkeiten, um einen Aktor eines Instruments schrittweise zu betätigen oder um das Instrument in zwei Dimensionen frei steuern zu können. Es ist daher bevorzugt, wenn die Steuereinheit mindestens ein Steuerelement in Art eines 2-Wege-Controllers oder 4-Wege-Controllers aufweist.

Je nach Art des Instruments können auch mehrere getrennte Steuerelemente vorgesehen sein, so beispielsweise ein 2-Wege-Controller oder ein 4-Wege-Controller zur Steuerung der Ausrichtung des Instruments sowie ein Betätigungsknopf zur Aktivierung des Instruments.

Die Steuereinheit ist erfindungsgemäß zur Anbringung oder zumindest zur Anlage an der Außenseite des Endoskopschlauchs ausgebildet. Sie verfügt entsprechend über einen Aufnahmebereich zur Aufnahme des Endoskopschlauchs oder über einen Anlagebereich zur definierten Anlage am Endoskopschlauch. Der Aufnahmebereich bzw. der Anlagebereich sind vorzugsweise zumindest abschnittsweise entsprechend einer Außenform des Endoskopschlauchs geformt, beispielsweise in Art einer Schalenform oder mit einer längserstreckten Nut.

Insbesondere verfügt die Steuereinheit vorzugsweise über einen mit dem Endoskop zusammenwirkenden Befestigungsmechanismus, um derart am Endoskopschlauch befestigt zu werden, dass der Benutzer die Hand vom Endoskopschlauch bzw. der Steuereinheit nehmen kann und diese am Endoskopschlauch in ihrer Position verbleibt. Zusätzlich oder alternativ kann ein mit dem Endoskop zusammenwirkenden Führungsmechanismus vorgesehen sein, insbesondere mit Rollen an der Steuereinheit, die am Endoskopschlauch anliegen.

Eine erfindungsgemäße Steuereinheit ermöglicht es, dass der Operateur jene Hand, mit der er den Endoskopschlauch führt, gleichzeitig auch nutzen kann, um mit ihr das transendoskopische Instrument zu steuern. Er kann daher alleine das Endoskop und das transendoskopische Instrument steuern und ist nicht darauf angewiesen, dass ein Assistent das transendoskopische Instrument steuert. Die entfallende Notwendigkeit eines Assistenten ermöglicht eine Reduzierung der Kosten der Operation. Zudem wird durch eine erfindungsgemäße Steuereinheit die Reaktionszeit bei Eingriffen verkürzt, da der Operateur direkt und ohne Verzögerung auf situative Anforderungen reagieren kann. Die unmittelbare Abstimmung der Steuerung der Endoskopspitze und des Instruments aufeinander wird durch die erfindungsgemäße Steuereinheit ermöglicht.

Grundsätzlich sind Bauweisen denkbar, bei denen ein eigenständiger Befestigungsmechanismus nicht erforderlich ist. Beispielsweise kann die Steuereinheit lose mit dem Endoskopschlauch verbunden sein oder nur an diesem anliegen, beispielsweise mittels einer passend dimensionierten Aufnahmenut für den Endoskopschlauch. Die Verbindung der Steuereinheit mit dem Endoskopschlauch in Schlaucherstreckungsrichtung wird bei einer solchen Gestaltung erst durch die Hand des Operateurs hergestellt, die die Steuereinheit und den anliegenden Endoskopschlauch gemeinsam umschließt.

Bevorzugt ist allerdings eine Gestaltung mit einem Befestigungsmechanismus, mittels dessen die Steuereinheit fest mit dem Endoskopschlauch verbunden wird. Dieser gestattet es, dass der Operateur die Hand von der Steuereinheit nehmen kann und diese hierdurch nicht zu Boden fällt, sondern am Endoskopschlauch verbleibt.

Vorzugsweise ist der Befestigungsmechanismus derart ausgestaltet, dass die Steuereinheit in definierter und gleichbleibender Position und Ausrichtung am Endoskopschlauch angebracht werden kann, so dass der Operateur alleine über das Greifen der Steuereinheit in die Lage versetzt wird, mittelbar den Endoskopschlauch zu steuern, insbesondere also den Endoskopschlauch zu drehen oder in den Körper des Patienten weiter einzuschieben oder aus diesem herauszuziehen.

In der Praxis hat es sich als zweckmäßig erwiesen, wenn der Befestigungsmechanismus in Erstreckungsrichtung des Endoskopschlauchs eine Kraft von mindestens 2 N, insbesondere von mindestens 5 N, von der Steuereinheit auf den Endoskopschlauch übertragen kann. 5 N reichen üblicherweise aus, um mittels der Steuereinheit den Endoskopschlauch zu bewegen, ohne dass die Steuereinheit am Endoskopschlauch entlanggleitet. Bevorzugt wird eine noch stärkere Verbindung, über die Kräfte von mehr als 10 N übertragen werden können.

Um Torsionsbewegung zu ermöglichen und damit die Endoskopspitze im Körper zu drehen, ist der Befestigungsmechanismus vorzugsweise derart ausgebildet, dass ein Torsionsmoment von mindestens 2 Nm, insbesondere von mindestens 4 Nm oder bevorzugt mindestens 8 Nm, über den Befestigungsmechanismus von der Steuereinheit auf den Endoskopschlauch übertragbar ist. Ein solches Torsionsmoment reicht üblicherweise aus, um den Endoskopschlauch im Körper des Patienten um die Erstreckungsrichtung des Endoskopschlauchs drehen zu können.

Der Befestigungsmechanismus kann zur dauerhaften Befestigung der Steuereinheit am Endoskopschlauch ausgebildet sein. Hierunter wird eine Befestigung verstanden, die im Laufe einer Operation üblicherweise nicht gelöst wird, sondern erst nach Abschluss der Operation. Ein solcher Befestigungsmechanismus kann als vergleichsweise schwer lösbarer Befestigungsmechanismus gestaltet sein, insbesondere derart, dass ein Lösen nur über eine Kraftbeaufschlagung einer die Freigabe bewirkenden Betätigungsfläche möglich ist, die vom Operateur üblicherweise nicht während der Operation berührt wird, so dass ein versehentliches Lösen unwahrscheinlich ist.

Der Befestigungsmechanismus kann jedoch auch gezielt derart ausgestaltet sein, dass er schnell und auch im Laufe der Operation gelöst werden kann. Um dies zu erreichen, wird eine Betätigungsfläche zum Lösen des Befestigungsmechanismus vorzugsweise in leicht zugänglicher Art an der Steuereinheit vorgesehen. Insbesondere kann in diesem Falle auch vorgesehen sein, dass der Befestigungsmechanismus eine Federeinrichtung umfasst, deren Federkraft dahingehend wirkt, dass sie die Steuereinheit am Endoskopschlauch klemmend festlegt, wobei über die Betätigungsfläche eine Auslenkung eines Teils des Befestigungsmechanismus gegen die Kraft der Feder bewirkt wird, um die klemmende Verbindung kurzzeitig zu lösen.

Ein Befestigungsmechanismus, der zum schnellen Lösen ausgebildet ist, kann von Vorteil sein, da der Operateur hierdurch gleichsam umgreifen kann. Wenn der Endoskopschlauch weiter in den Körper des Patienten eingeschoben wird und die Steuereinheit sich somit dem Eintrittspunkt des Endoskopschlauchs nähert, kann der Operateur mit einer kurzen Betätigung der Betätigungsfläche die Befestigung temporär lösen, die Steuereinheit entlang des Endoskopschlauchs zurückziehen und dann die Betätigungsfläche wieder loslassen, so dass über die Federeinrichtung der befestigte Zustand versetzt zur vorherigen Befestigungsposition wieder hergestellt wird. Die Federeinrichtung selbst ist vorzugsweise als Druckfeder ausgebildet. Dies bedeutet, dass sie in einer Federrichtung komprimiert wird, um die Befestigung der Steuereinheit am Endoskopschlauch zu lösen.

Der Befestigungsmechanismus, mittels dessen die Steuereinheit am Endoskopschlauch befestigt ist, verfügt vorzugsweise über mindestens eine erste Haltefläche und mindestens eine gegenüberliegende zweite Haltefläche zur beidseitigen Anlage am Endoskopschlauch. Die Halteflächen ermöglichen gemeinsam eine klemmende Verbindung. Es kann vorteilhaft sein, mindestens eine der Halteflächen aus einem elastischen und vorzugsweise gummiartigen Material zu bilden, um die Haftreibung zwischen Halteflächen und Endoskopschlauch und somit die übertragbaren Kräfte bzw. Momente zu vergrößern. Weiterhin kann es sinnvoll sein, eine oder beide Halteflächen mit einer Nut zu versehen, um die Anlagefläche am Endoskopschlauch zu vergrößern und um zu gewährleisten, dass der Endoskopschlauch relativ zur Haltefläche in einer definierten Position verbleibt.

Es ist bevorzugt, wenn der Befestigungsmechanismus beidseitig des Endoskopschlauchs jeweils mindestens zwei Halteflächen aufweist, die bezogen auf eine Erstreckungsrichtung des Endoskopschlauchs voneinander beabstandet eine klemmende Verbindung mit dem Endoskopschlauch gestatten. Der Abstand zweier Halteflächen auf der gleichen Seite des Endoskopschlauchs beträgt vorzugsweise mindestens 20 mm, insbesondere 30 mm oder mehr.

Die in Erstreckungsrichtung des Endoskopschlauchs mehreren Halteflächen führen dazu, dass die Steuereinheit besonders sicher am Endoskopschlauch befestigt ist und nicht zu befürchten ist, dass die Steuereinheit um eine Querachse zum Endoskopschlauch ungewünscht verkippt.

Gegenüber einer einheitlichen Anlagefläche, die sich entlang des Endoskopschlauchs erstreckt, beispielsweise ebenfalls über eine Strecke von 30 mm, ist die Gestaltung mit beabstandeten Halteflächen von Vorteil, da der Endoskopschlauch zwischen den Halteflächen noch eine Restbeweglichkeit aufweisen kann und somit der Endoskopschlauch durch die Steuereinheit nicht über eine längere Teilstrecke hinweg ausgesteift wird.

Die Steuereinheit verfügt vorzugsweise über eine Haupteinheit, an der mindestens ein Steuerelement vorgesehen ist, also beispielsweise der genannte 4-Wege-Controller. Vorzugsweise ist die Gesamtheit der elektronischen Komponenten der Steuereinheit in der Haupteinheit vereint.

Die Steuereinheit verfügt weiterhin vorzugsweise über einen demgegenüber beweglichen Befestigungsträger, wobei auf Seite der Haupteinheit und auf Seite des Befestigungsträgers jeweils mindestens eine Haltefläche vorgesehen ist.

Der klemmende Zustand zur Befestigung der Steuereinheit am Endoskopschlauch wird durch eine Relativbewegung von Haupteinheit und Befestigungsträger bewirkt. Der Befestigungsträger ist vorzugsweise definiert gegenüber der Haupteinheit beweglich, insbesondere linearbeweglich oder schwenkbeweglich. Im Falle von Schwenkbeweglichkeit ist der Befestigungsträger vorzugsweise um eine Schwenkachse gegenüber der Haupteinheit verschwenkbar, die im Wesentlichen orthogonal zur Aufnahme für den Endoskopschlauch ausgerichtet ist.

Wenn eine Federeinrichtung der oben beschriebenen Art vorgesehen ist, wirkt dies vorzugsweise zwischen der Haupteinheit und dem Befestigungsträger, insbesondere dahingehend, dass durch die Federkraft ein Zwischenraum zwischen Haupteinheit und Befestigungsträger, in dem der Endoskopschlauch angeordnet ist, verkleinert wird. Der Befestigungsträger kann dementsprechend manuell kraftbeaufschlagt werden, um die Federeinrichtung zu spannen und die Verbindung zwischen Steuereinheit und Endoskopschlauch zu lösen.

Die Haupteinheit und der Befestigungsträger können über zusammenwirkende Rasteinrichtungen verfügen, durch die beim Festklemmen der Steuereinheit am Endoskopschlauch die Haupteinheit und der Befestigungsträger miteinander verrasten und somit gegen Trennung gesichert sind. Der klemmende Zustand wird somit gleichsam automatisch gesichert, wenn die Haupteinheit der Steuereinheit und der Befestigungsträger nach Einlegen des Endoskopschlauchs aufeinander zugedrückt werden.

Insbesondere kann eine Mehrzahl von Rastpositionen durch die Rasteinrichtungen definiert sein. Dies bedeutet, dass die Haupteinheit und der Befestigungsträger nicht nur in einer definierten Relativstellung verrasten, sondern in mehreren möglichen Stellungen. Insbesondere können drei oder mehr Rastpositionen vorgesehen sein, insbesondere durch eine Rastfläche mit mindestens drei benachbarten Erhebungen, die jeweils geeignet sind, den Rastzustand herzustellen. Vorzugsweise sind an beiden Rasteinrichtungen mehrere benachbarte Erhebungen vorgesehen. Eine Rasteinrichtung mit mehreren Rastpositionen gestattet es, die Klemmkraft am Endoskopschlauch zu variieren bzw. die Steuereinheit mit Endoskopschläuchen unterschiedlichen Durchmessers zu verwenden.

Die Steuereinheit weist vorzugsweise eine ergonomische Formgebung auf, die spezifisch zur Führung der Steuereinheit mit der rechten Hand oder der linken Hand ausgebildet ist. Dies bedeutet, dass die Steuereinheit nicht symmetrisch ausgebildet ist, sondern für das Greifen mit der linken oder rechten Hand. Insbesondere kann sie dafür ausgebildet sein, mit der rechten Hand bedient zu werden.

Die für eine bestimmte Hand vorgesehene Bauform geht vorzugsweise insbesondere damit einher, dass Auflageflächen an der Steuereinheit spezifisch für die Anlage der rechten oder linken Hand vorgesehen sind, auf der gegenüberliegenden Seite der Steuereinheit jedoch nicht vorgesehen sind. Weiterhin ist vorzugsweise vorgesehen, dass das mindestens eine Bedienelement entsprechend der Anpassung an die linke oder die rechte Hand an der Steuereinheit angeordnet ist, insbesondere also von der Symmetrie abweichend schräggestellt oder verschoben zu einer Mittellinie.

Wenn die Steuereinheit einen 4-Wege-Controller oder einen 2-Wege-Controller aufweist, so ist dieser vorzugsweise an der Steuereinheit derart vorgesehen, dass er bei bestimmungsgemäßem Fassen der Steuereinheit mit der Hand im Bereich des Daumens angeordnet ist. Vorzugsweise ist dieser Controller in Richtung des Daumens verkippt angebracht.

Im Falle eines 4-Wege-Controllers weist dieser vorzugsweise einen in Richtung zweier Achsen beweglichen Steuerabschnitt auf, wobei der Steuerabschnitt vorzugsweise in einer Neutralstellung mit dem Endoskopschlauch einen Winkel größer 0° und kleiner 90° einschließt, insbesondere einen Winkel größer 20° und kleiner 70°, um ein ergonomisches Bedienen zu erleichtern.

Eine besondere Form einer erfindungsgemäßen Steuereinheit sieht vor, dass die Steuereinheit eine seitliche Ausnehmung aufweist, in die der Endoskopschlauch von der Seite der Länge nach eingeschoben werden kann, so dass er nach dem Einschieben vorne und hinten aus der Steuereinheit hinausragt. Eine solche Bauweise ist schnell und intuitiv anzubringen.

Eine weitere vorteilhafte Gestaltung sieht vor, dass die Steuereinheit eine Haltehilfe in Form eines Fingerlochs aufweist, in das der Bediener beim Greifen einen Finger einführt. Ein solches Fingerloch führt zu einem besonders guten Halt und gestattet es, die Steuereinheit besonders präzise zusammen mit dem Endoskopschlauch vor- und zurückschieben zu können. Insbesondere bei Steuereinheiten ohne Befestigungsmechanismus ist es von Vorteil, wenn ein solches Fingerloch vorgesehen ist.

Eine weitere Bauweise der Steuereinheit sieht vor, dass ein Führungsmechanismus vorgesehen ist, insbesondere mit Rollen an der Steuereinheit, die am Endoskopschlauch anliegen. Die Steuereinheit kann mittels eines solchen Führungsmechanismus leichtgängig entlang des Endoskopschlauchs verlagert werden. Vorzugsweise kann der Operateur mindestens eine Rolle blockieren, um die Steuereinheit an einer bestimmten Stelle des Endoskopschlauchs zu arretieren.

Neben der Steuereinheit betrifft die Erfindung auch ein Endoskopzubehör, welches ein transendoskopisches Instrument zur Durchführung durch den Arbeitskanal eines Endoskops umfasst sowie eine Steuereinheit oben beschriebener Art. Das transendoskopische Instrument verfügt über mindestens einen elektromechanisch betätigbaren Aktor, der je nach Art des Instruments bei Benutzung in der Nähe der Endoskopspitze vorgesehen ist. Es kann sich beispielsweise um einen auf dem metallischen Formgedächtnis basierenden Aktor handeln, der im Arbeitskanal des Endkoskops in Nähe der Endoskopspitze angeordnet ist. Vorzugsweise erstreckt sich eine Steuerleitung durch den Arbeitskanal des Endoskops bis zu diesem Aktor. Der Aktor kann jedoch auch extern vorgesehen sein, beispielsweise an einem stationären Steuergerät. Die hier erzeugte Bewegung wird dann über mechanische Kopplungselemente wie Bowdenzüge an das Werkzeug des Endoskopzubehörs übertragen.

Insbesondere kann das transendoskopische Instrument ein Werkzeug umfassen, welches mittels des elektromechanisch betätigbaren Aktors gegenüber einer stirnseitigen Öffnung des Arbeitskanals verlagerbar ist, insbesondere ausrichtbar ist. Diese Verlagerung kann bei einer erfindungsgemäßen Gestaltung mittels der Steuereinheit gesteuert werden. Das Werkzeug kann zusätzlich gegenüber einem im Arbeitskanal beweglichen Instrumentenstrang zusätzlich aktorisch oder manuell verlagerbar sein.

Das Werkzeug kann beispielsweise als bewegliches Messer insbesondere für die endoskopische Submukosadissektion ausgebildet sein und dazu dienen, die Mukosaschicht gezielt von der Submukosa zu lösen. Die Steuereinheit ermöglicht hier die präzise Steuerung der Schneidbewegung, beispielsweise durch eine kontrollierte Winkeländerung (Auslenkung)) oder durch eine kontrollierte Vor- und Zurückbewegung des Messers.

Das Werkzeug kann auch als Hämostasewerkzeug ausgebildet sein und wird zur Blutstillung eingesetzt, beispielsweise durch thermische Koagulation oder mechanischen Druck.Über die Steuereinheit kann der Operateur zielgerichtet durch die Winkelanpassung des Instrumentes dieses richtig positionieren und/oder die Aktivierung der Hämostasefunktion auslösen.

Das Werkzeug ist weiterhin als endoskopischer Clipapplikator ausgebildet, der zur Gewebeapproximation oder Blutstillung durch das Setzen von Clips dient. Die Steuereinheit erlaubt in einem solchen Falle die kontrollierte Platzierung und das gezielte Freisetzen der Clips an der gewünschten Stelle.

Das Werkzeug kann auch als Sonde ausgebildetsein, insbesondere als APC-Sonde für Argon-Plasma-Koagulation, als RFA-Sonde für Radiofrequenzablation oder als EUS-Sonde für endoskopischen Ultraschall. Die Steuereinheit kann hierbei die Aktivierung der Energieabgabe, die Intensitätsregulierung oder die Positionierung der Sonde steuern.

Ist das Werkzeug als Injektionsnadel ausgebildet, so dient es zur gezielten Applikation von Flüssigkeiten, beispielsweise für die Submukosainjektion. Die Steuereinheit ermöglicht die präzise Auslenkungen der Nadel und/oder Vorschieben der Nadel und/oder das kontrollierte Injizieren des Mediums.

Das Werkzeug kann weiterhin als Nahtwerkzeug dienen und insbesondere zur endoskopischen Gewebeapproximation eingesetzt werden. Über die Steuereinheit kann die Nahtführung, das Durchziehen des Fadens und das Knoten der Naht gesteuert werden.

Ist das Werkzeug als Biopsiezange ausgebildet, so dient es zur Entnahme von Gewebeproben. Die Steuereinheit ermöglicht die gezielte Öffnung und Schließung der Zange und ggf. das kontrollierte Vorschieben und Zurückziehen im Arbeitskanal. Das Werkzeug kann auch als Schlinge ausgebildet sein und zur Entfernung von Gewebestrukturen eingesetzt werden. Die Steuereinheit erlaubt in einem solchen Falle die kontrollierte Platzierung der Schlinge um die zu entfernende Gewebestruktur. Ähnlich ist die Funktion, wenn das Werkzeug als endoskopische Schere ausgebildet ist, die das präzise Schneiden von Gewebestrukturen oder Fremdkörpern mittels der Steuereinheit ermöglicht. Die Steuereinheit erlaubt hierbei insbesondere eine gezielte Steuerung der Positionierung sowie das kontrollierte Öffnen und Schließen der Scherenblätter.

Das Werkzeug kann auch als Fangnetz ausgebildet sein und zur sicheren Erfassung und Entfernung von Gewebestücken oder Fremdkörpern dienen. Über die Steuereinheit kann das Netz geöffnet, geschlossen und im Körperinneren positioniert werden.

Ist das Werkzeug als bewegliches Papillotom ausgebildet, so wird es zur gezielten Inzision der Papille eingesetzt, beispielsweise bei endoskopischen Eingriffen an den Gallen- oder Pankreasgängen. Die Steuereinheit ermöglicht in einem solchen Falle eine präzise Steuerung der Schneidebewegung und der Platzierung.

Das Werkzeug kann auch als bewegliches Cholangioskop ausgebildet sein und durch ein übergeordnetes Endoskop als transendoskopisches Werkzeug eingeführt werden. Es dient zur gezielten Navigation und Therapie innerhalb der Gallengänge. Die Steuereinheit kann hierbei das Vorschieben, Zurückziehen und gegebenenfalls die Aktivierung zusätzlicher Funktionen steuern. Das Werkzeug kann weiterhin als Ballondilatator ausgebildet sein und wird primär zur Aufdehnung von Stenosen in verschiedenen Körperregionen eingesetzt. Über die Steuereinheit kann die Expansion des Ballons präzise gesteuert und an die anatomischen Gegebenheiten angepasst werden.

Wie aus den voranstehenden Anwendungsfeldern zu ersehen ist, ist es in den meisten Fällen hilfreich, zwei unterschiedliche Funktionen durch die Steuerelemente bereitzustellen, nämlich üblicherweise einerseits die Positionierung des eigentlichen Werkzeugs und andererseits eine Betätigung nach zuvor erfolgter Positionierung. Es ist daher bevorzugt, wenn ein einziges Steuerelement diese unterschiedlichen Funktionen in sich vereint. Demgegenüber ist es in der Praxis von Vorteil, wenn zwei Steuerelemente vorgesehen sind, nämlich eines zum Steuern der Position oder Ausrichtung des Werkzeugs und eines zur Betätigung. Durch die Trennung der Steuerelemente wird ermöglicht, dass der Operateur nach erfolgter Positionierung des Werkzeugs das entsprechende Steuerelement, wie beispielsweise einen 4-Wege-Controller, loslassen kann und dann stattdessen ein zweites Steuerelement betätigt, welches nur der Betätigung des jeweiligen Werkzeugs dient.

Die Anordnung von Steuerelementen an der Steuereinheit kann insbesondere vorsehen, dass im Bereich des Daumens und/oder Zeigefingers Steuerelemente vorgesehen sind.

Die bestimmungsgemäß am Endoskopschlauch vorgesehene Steuereinheit kommuniziert mit dem Instrument und insbesondere dessen Aktoren bzw. einer vorgeschalteten Steuerelektronik, um die von der Steuereinheit stammenden Signale in Aktorbetätigung umzusetzen. Grundsätzlich kann diese Kommunikation drahtgebunden erfolgen. In diesem Falle ist ein Steuerkabel vorgesehen, das vorzugsweise parallel zum Endoskopschlauch von der Steuereinheit zum Steuerkopf des Endoskops verläuft. Das Steuerkabel kann mittels Befestigungsbändern oder -clips am Endoskopschlauch befestigt sein.

Bevorzugt wird allerdings eine drahtlose Gestaltung der Übertragung. Die Steuereinheit weist zu diesem Zweck vorzugsweise einen Funksender auf, zu dem korrespondierend ein Funkempfänger am Instrument vorgesehen ist, insbesondere als Teil von dessen Steuerelektronik. Der Funkempfänger kann auch an einem externen Steuergerät vorgesehen sein, an dem das Instrument elektrisch oder mechanisch angeschlossen ist.

Der Funksender und der Funkempfänger können gemäß einem üblichen Funkstandard ausgebildet sein, beispielsweise WiFi oder Bluetooth, gegebenenfalls besondere Varianten hiervon für den medizinischen Sektor. Funksender und Funkempfängersind gemäß den jeweiligen Spezifikationen miteinander gekoppelt, um Daten auszutauschen. Vorzugsweise ist diese Kopplung permanent und durch den Verwender nicht bestimmungsgemäß aufzuheben oder eine neue Kopplung herstellbar. Auf diesem Wege werden Schwierigkeiten vermieden, die sich durch wechselnde Kopplungen ergeben könnten. Zwar führt eine feste Kopplung dazu, dass die Steuereinheit zusammen mit dem Werkzeug zu entsorgen ist oder zum Recycling gegeben wird. Der Relevanz einer problemlosen Kopplung überwiegt im medizinischen Bereich jedoch, so dass es vertretbar ist, dass das transendoskopische Instrument und die Steuereinheit als Einwegkomponenten ausgebildet sind.

Es ist jedoch auch eine wahlfreie Kopplung möglich. Dies kann dem Zweck dienen, ein gleichbleibendes Steuergerät mit mehreren verschiedenen Werkzeugen während einer Operation zu verwenden. Das Steuergerät kann zu diesem Zweck dafür ausgebildet sein, mit mehreren Instrumenten koppelbar zu sein bzw. zwischen gekoppelten Instrumenten umschalten zu können.

Vorzugsweise ist vorgesehen, dass das transendoskopische Instrument eine Steuerelektronik aufweist, in der eine eindeutige Geräte-ID der Steuereinheit hinterlegt ist, wobei die Steuerelektronik dafür ausgebildet ist, eine Steuerung des Aktors nur zu ermöglichen, wenn darauf gerichtete Steuerbefehle von der Steuereinheit mit der hinterlegten Geräte-ID stammen.

Die Kommunikation des transendoskopischen Instruments und der Steuereinheit erfolgt vorzugsweise über einen kryptographisch geschützten Kanal.

Die Erfindung betrifft weiterhin auch ein Endoskopsystem als Ganzes. Das Endoskopsystem verfügt über ein Endoskop mit einem flexiblen Endoskopschlauch, in dem ein Arbeitskanal für ein Endoskopzubehör vorgesehen ist. Das Endoskopzubehör ist in oben bezeichneter Art ausgebildet.

Ein solches Endoskopsystem kann insbesondere als Set ausgebildet sein, welches ein Instrument der beschriebenen Art umfasst und hiervon getrennt ein Endoskop, wobei das Instrument und das Endoskop in oben bezeichneter Weise zusammenwirken können.

Es ist jedoch auch eine integrierte Gestaltung denkbar, beispielsweise eine solche, bei der ein Steuerkabel fest in den Endoskopschlauch integriert ist oder eine solche, bei der die Steuereinheit fest und werkzeuglos unlösbar am Endoskopschlauch angebracht ist.

Sofern die Steuereinheit mit einem durch den Verwender zu bedienenden Befestigungsmechanismus versehen ist, ist im Falle eines Endoskopsystems als Ganzes eine enge Abstimmung zwischen dem Befestigungsmechanismus und dem Endoskopschlauch möglich.

Die Steuereinheit des Endoskopzubehörs ist vorzugsweise derart an den flexiblen Endoskopschlauch angepasst, dass über den Befestigungsmechanismus eine Schubkraft in Erstreckungsrichtung des Endoskopschlauchs von mindestens 2 N, insbesondere von mindestens 5 N, von der Steuereinheit auf den Endoskopschlauch übertragbar ist. Ein Torsionsmoment von mindestens 2 Nm, insbesondere von mindestens 4 Nm, ist vorzugsweise von der Steuereinheit auf den Endoskopschlauch übertragbar.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt eine Operationssituation, in der ein erfindungsgemäßes Endoskopsystem Anwendung findet.
Fig. 2 zeigt eine Endoskopspitze und ein hieran vorgesehenes transendoskopisches Instrument.
Fig. 3 bis 8 zeigen eine Variante einer Steuereinheit zur Steuerung des transendoskopischen Instruments während der Operation, wobei die Fig. 3 und 4 die Steuereinheit während der Befestigung an einem Endoskopschlauch und Fig. 5 und 6 den befestigten Zustand zeigen. In Fig. 7 ist ein schematischer Querschnitt der Steuereinheit gezeigt. Fig. 8 verdeutlicht die Verwendung der Steuereinheit.
Fig. 9A bis 9B zeigen einen alternativen Befestigungsmechanismus zur Anbringung des Steuersystems am Endoskopschlauch in drei verschiedenen Stadien.
Fig. 10 bis 13 zeigen eine weitere Variante einer Steuereinheit zur Steuerung des transendoskopischen Instrument während der Operation.
Fig. 14 zeigt eine Steuereinheit zur Steuerung des transendoskopischen Instrument während der Operation, welche mittels Rollen am Endoskopschlauch geführt ist.

### DETAILLIERTE BESCHREIBUNG DER AUSFÜHRUNGSBEISPIELE

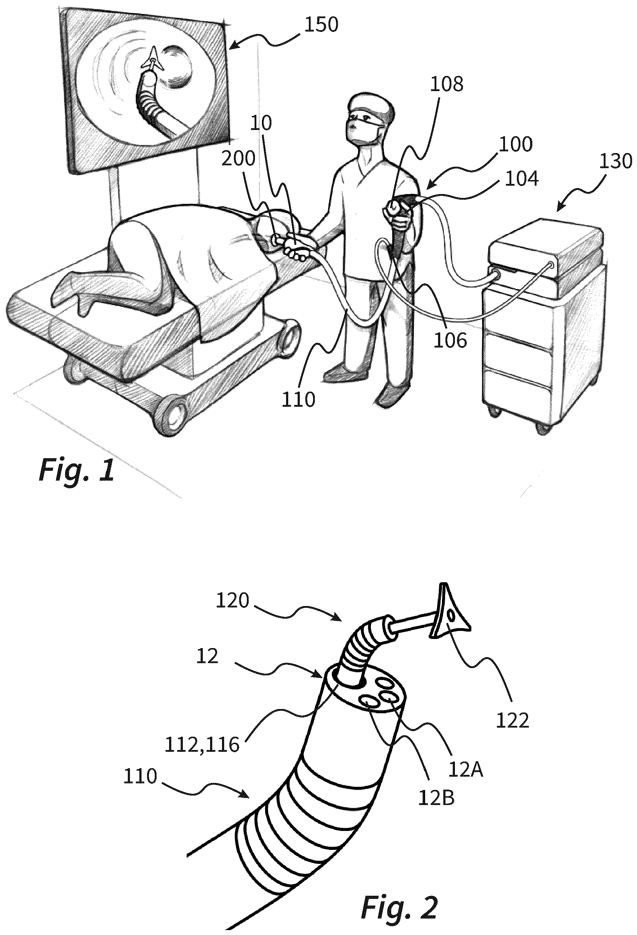

Fig. 1 zeigt in Gesamtdarstellung eine Operationssituation, in der ein erfindungsgemäßes Endoskopsystem 100 bestimmungsgemäß Anwendung findet.

Während einer endoskopischen Operation wird das Endoskopsystem 100 vom Operateur manuell geführt. Der Operateur führt einen Steuerkopf 104 mit seiner linken Hand. Am Steuerkopf 104 ist ein flexibler Endoskopschlauch 110 angebracht, der vom Operateur mit der rechten Hand geführt wird. Der Endoskopschlauch 110 ist in den Körper des Patienten eingeführt. Im vorliegenden Beispiel erfolgt dies über den Mund als Eintrittspunkt 200.

Der Operateur steuert die Endoskopspitze 12 am distalen Ende des Endoskopschlauchs einerseits mittels der rechten Hand, indem er den Endoskopschlauch 110 manuell kraftbeaufschlagt, beispielsweise vorschiebt und damit tiefer in die Speiseröhre eines Patienten eindringt, oder tordiert und damit auch die Ausrichtung der Endoskopspitze ändert. Andererseits steuert der Operateur die Endoskopspitze 12 mittels Stellrädern 108 am Steuerkopf 104 und über Bowdenzüge, die innerhalb des Endoskopschlauchs 110 geführt sind.

Seitlich am Steuerkopf 104 ist ein Instrumentenzugang 106 vorgesehen, durch den hindurch ein transendoskopisches Instrument 120 in einen Arbeitskanal 112 des Endoskopschlauchs 110 eingeführt wird.

Auf einem Bildschirm 150 wird ein Kamerabild einer Kamera 12A an der Endoskopspitze 12 angezeigt, anhand dessen der Operateur die Operation durchführt. Fig. 2 zeigt, dass an der Endoskopspitze 12 neben der Kamera 12A und einer Lichtquelle 12B der Ausgang des Arbeitskanals 112 vorgesehen ist.

Das Instrument 120 erstreckt sich aus der Endoskopspitze 12 heraus und kann eine längs ausgerichtete oder gekrümmte Stellung einnehmen. Hierdurch wird ermöglicht, dass das Instrument 120 bzw. dessen Werkzeug 122 relativ zur ebenfalls beweglichen Endoskopspitze 12 bewegt werden kann.

Die Steuerung des Werkzeugs 122 erfolgt mittels einer als Handteil ausgebildeten Steuereinheit 10, welche vom Operateur mit seiner rechten Hand bedient wird. Die rechte Hand umgreift die Steuereinheit 10, die ihrerseits am Endoskopschlauch 110 befestigt ist, so dass der Operateur den Endoskopschlauch 110 über die Steuereinheit 10 in etwa so führen kann, wie es beim direkten Führen des Endoskopschlauchs 110 der Fall wäre. Je nach Ausgestaltung kann der Operateur die Steuereinheit 10 auch kurzzeitig lösen, um sie entlang des Endoskopschlauchs 110 verschieben zu können.

Mögliche Varianten für die Steuereinheit 10 sind auf den folgenden Seiten dargestellt.

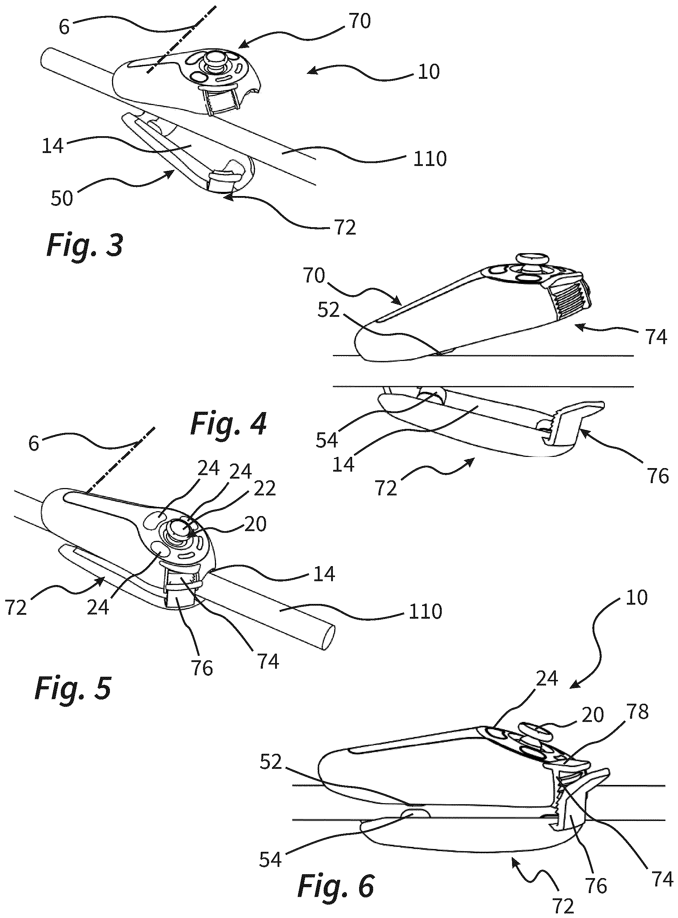

Die Fig. 3 bis 8 zeigen eine erste Variante.

In Fig. 3 und 4 ist erkennbar, dass die hier dargestellte Steuereinheit 10 zwei gegeneinander bewegliche Teileinheiten aufweist, nämlich die Haupteinheit 70 und einen Befestigungsträger 72. Der Befestigungsträger 72 ist um die Schwenkachse 6 schwenkbar an der Haupteinheit 70 angebracht. Gemeinsam definieren der Befestigungsträger 72 und die Haupteinheit 70 einen Aufnahmebereich 14, der zur Aufnahme des Endoskopschlauchs 110 vorgesehen ist. Das Scharnier, mittels dessen der Befestigungsträger 72 schwenkbar an der Haupteinheit 70 angebracht ist, ist einseitig vorgesehen, so dass von der gegenüberliegenden Seite der Endoskopschlauch 110 eingeschoben werden kann. An den zum Aufnahmebereich 14 weisenden Seiten der Haupteinheit 70 und des Befestigungsträgers 72 sind Halteflächen 52, 54 vorgesehen, die aus weichem, elastischem Material gebildet sind und dadurch den Endoskopschlauch 110 sicher halten können.

Die Haupteinheit 70 und der Befestigungsträger 72 verfügen über Rasteinrichtungen 74, 76, die an einem vorderen Ende seitlich angebracht sind. Die Rasteinrichtungen 74, 76 umfassen vorliegend jeweils Rastleitern mit einer Vielzahl von Rastkanten.

Die Rasteinrichtungen 74, 76 dienen dem Zweck, die Haupteinheit 70 und den Befestigungsträger 72 in zugeklapptem Zustand an den Endoskopschlauch 110 anzupressen und dadurch eine stabile Klemmverbindung herzustellen.

Die Fig. 5 und 6 zeigen diesen Zustand, in dem die Steuereinheit 10 am Endoskopschlauch 110 festgeklemmt ist. Es ist zu erkennen, dass die Rasteinrichtungen 74, 76 verrastet sind und ohne manuelles Lösen über eine Freigabelasche 78 nicht mehr lösbar sind. Durch die Rastleitern sind die Rasteinrichtungen 74, 76 in unterschiedlichen Relativstellungen verrastbar, so dass die Steuereinheit 10 mit Endoskopschläuchen unterschiedlicher Dicke verwendbar ist.

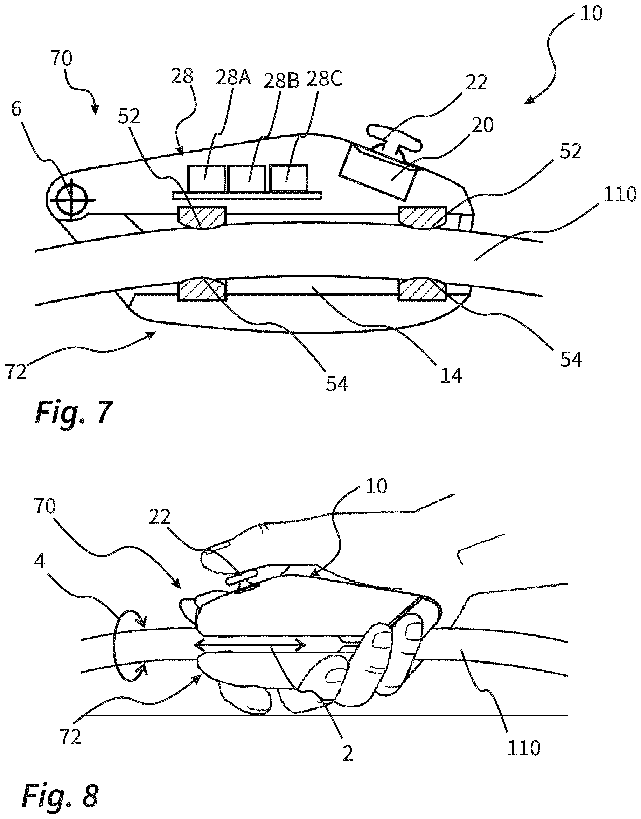

Fig. 7 zeigt die Steuereinheit 10 in einer schematischen geschnittenen Ansicht. Der 4-Wege-Controller 20 ist mit einer Steuerelektronik 28 verbunden, die unter anderem eine Batterie 28A, einen Prozessor 28C sowie ein Funkmodul 28B umfasst. Der Fig. 7 ist auch zu entnehmen, dass es zweckmäßig ist, die Halteflächen 52 bzw. 54 ausreichend weit voneinander zu beabstanden, so dass der Endoskopschlauch 110 auch innerhalb des Aufnahmebereichs 14 eine gekrümmte Stellung einnehmen kann.

Fig. 8 verdeutlicht, wie der Operateur die Steuereinheit nutzen kann. Der Operateur umgreift die Steuereinheit 10, so dass sein Daumen im Bereich des 4-Wege-Controllers 20 angeordnet ist und auf dessen beweglichen Steuerabschnitt 22 ruht und diesen gut bedienen kann. Zudem ist der Zeigefinger im Bereich der Rasteinrichtungen 74, 76 angeordnet, so dass diese einfach gelöst werden können, um die Steuereinheit 10 entlang des Endoskopschlauchs 110 zu verschieben und dann wieder verrasten zu können.

Die Klemmverbindung zwischen dem Endoskopschlauch 110 und der Steuereinheit 10 ist vorzugsweise derart gestaltet, dass Schubkräfte 2 von mehr als 5 Newton und/oder Torsionsmomente 4 von mehr als 4 Nm von der Steuereinheit 10 auf den Endoskopschlauchs 110 und damit auf die Endoskopspitze 12 übertragen werden können.

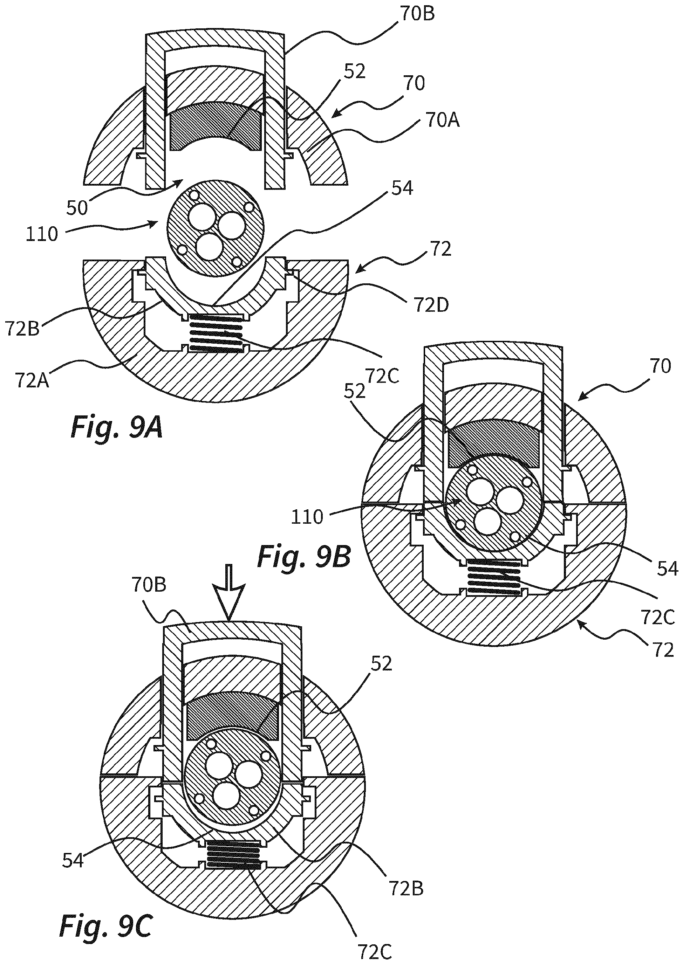

Fig. 9A bis 9C zeigen in schematischer Darstellung eine Variante zur Steuereinheit der Fig. 3 bis 8. Gezeigt ist diese Steuereinheit 10 in einer Schnittebene ihres Befestigungsmechanismus.

Fig. 9A zeigt den Zustand vor der Befestigung der Steuereinheit 10 am Endoskopschlauch 110. Die Haupteinheit 70 und der Befestigungsträger 72 sind aufgeklappt und daher in der Schnittebene der Fig. 9A noch beabstandet. Es ist in Fig. 9A zu erkennen, dass der Befestigungsträger 72 eine Innenschale 72B und eine Außenschale 72A umfasst. Die Innenschale 72B ist in einer Vertiefung der Außenschale 72A geführt und wird durch eine Federeinrichtung 72C in eine obere Endlage gedrückt, bis Anschlagsflächen 72D der Innenschale 72B an korrespondierenden Halteflächen der Außenschale 72A anliegen.

Die Innenschale 72B bildet eine untere Haltefläche 54 für den Endoskopschlauch 110.

Der Befestigungsmechanismus 50 umfasst an der Haupteinheit 70 eine obere Haltefläche 52, die an einem elastischen Klemmblock vorgesehen ist. Zudem umfasst die Haupteinheit 70 neben einer Außenschale 70A einen Freigabedrücker 70B, dessen Funktion wird weiter erläutert.

Fig. 9B zeigt den verrasteten Zustand. Die Haupteinheit 70 und der Befestigungsträger 72 sind mittels Rastmitteln entsprechend der Fig. 3 bis 8 verrastet. Der Endoskopschlauch 110 ist nun zwischen den Halteflächen 52, 54 eingeklemmt und kann über die Steuereinheit 10 geführt werden. Die Federeinrichtung 72C drückt dauerhaft von unten gegen den Endoskopschlauch 110.

Fig. 9C zeigt einen temporär gelösten Zustand. Wenn der Operateur auf den Freigabedrücker 70B drückt, so drückt dieser die Innenschale 72B und mit ihr die Haltefläche 54 gegen die Kraft der Federeinrichtung 72C hinunter, während die Haupteinheit 70 und der Befestigungsträger 72 unverändert verrastet bleiben. In diesem Zustand kann die Steuereinheit 10 nun entlang des Endoskopschlauchs 110 verschoben werden, bis der Operateur den Freigabedrücker 70B loslässt, so dass die Feder 72C sich entspannen kann und dann wiederum den Endoskopschlauch 110 an der Steuereinheit 10 festlegt.

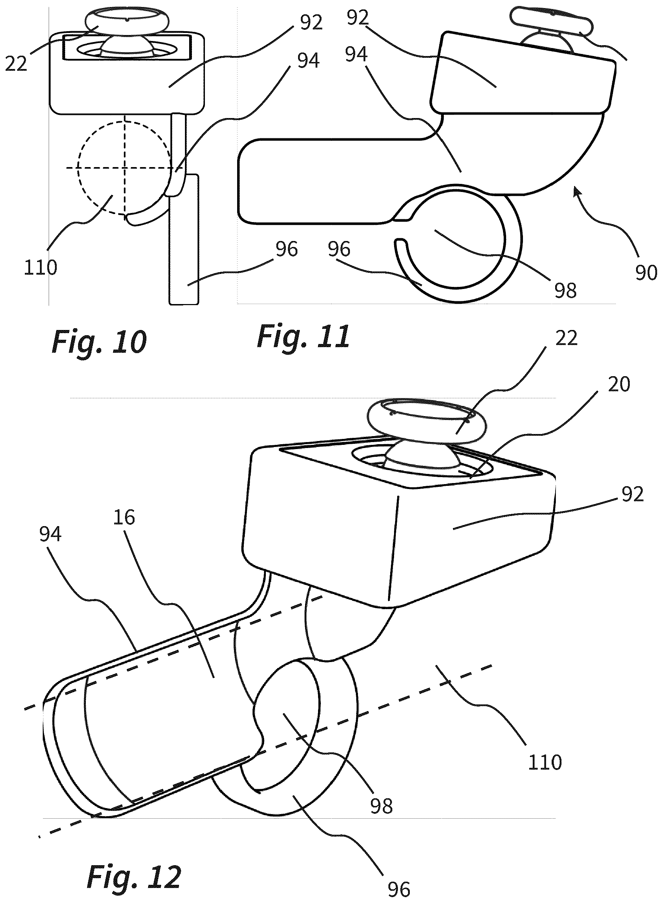

Die dritte Variante ist in Fig. 10 bis 13 gezeigt. Bei dieser dritten Variante ist kein Aufnahmeraum vorgesehen, innerhalb dessen der Endoskopschlauch 110 zwischen Klemmflächen gehalten ist. Stattdessen weist die Steuereinheit 10 gemäß Fig. 10 bis 13 einen mehrgliedrigen, aber in sich unbeweglichen Körper 90 auf. Dieser Körper 90 verfügt über einen Steuerabschnitt 92, dessen Oberseite angewinkelt ist und einen 4-Wege-Controller 20 umfasst. Unterhalb des Steuerabschnitts 92 schließt sich ein gewölbter Anlageabschnitt 94 an, dessen Innenseite einen Anlagebereich 16 definiert. Am Anlageabschnitt 94 wiederum ist ein Ringabschnitt 96 mit einem Fingerloch 98 vorgesehen.

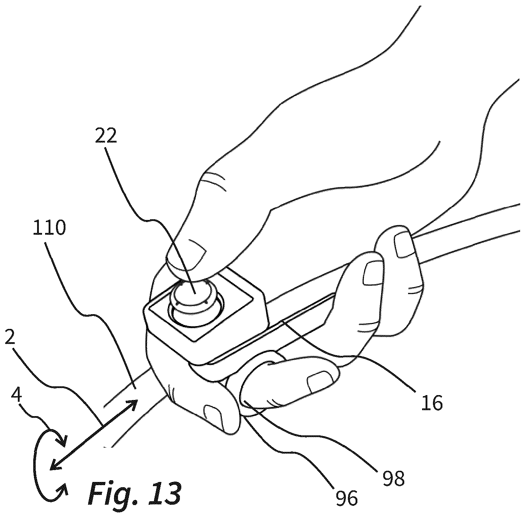

Fig. 13 verdeutlicht die Handhabung. Der Operateur umgreift den Körper 90 der Steuereinheit 10, wobei er die Mittelfinger durch das Fingerloch 98 steckt. Den Endoskopschlauch 110 führt er zwischen dem Anlagebereich 16 und seiner Handinnenfläche. Sobald der Operateur den Griff lockert, wird der Endoskopschlauch 110 nicht mehr gegen den Anlagebereich 16 gepresst und die Steuereinheit 10 lässt sich entlang des Endoskopschlauchs 110 verschieben und auch komplett vom Endoskopschlauch 110 trennen.

Drückt der Operateur allerdings den Endoskopschlauch 110 zwischen der Handinnenfläche und dem Anlagebereich fest, so kann er dann über die Steuereinheit 10 den Endoskopschlauch 110 in Längsrichtung mit einer Schu bkraft 2 oder einem Torsionsmoment 4 beaufschlagen, welches dann auf die Endoskopspitze 12 übertragen wird.

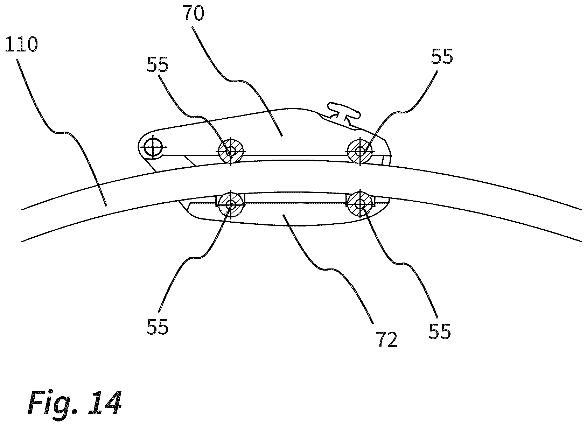

Fig. 14 zeigt eine weitere Bauweise, die eine Variante zur Gestaltung der Fig. 3 bis 8 darstellt. Wie anhand der Fig. 14 zu ersehen ist, sind hier keine festen Halteflächen 54, 56 vorgesehen, sondern Rollen 55, die einen Führungsmechanismus bilden. Die Steuereinheit 10 kann mittels der Rollen leichtgängig entlang des Endoskopschlauchs verlagert werden. In nicht dargestellter Weise ist vorzugsweise eine Blockiereinrichtung vorgesehen, mittels derer einzelne Rollen blockiert werden können, so dass die Steuereinheit 10 hierdurch ortsfest am Endoskopschlauch 110 gesichert ist.

## Patentansprüche

1. Steuereinheit (10) zur manuellen Steuerung eines transendoskopischen Instruments mit dem folgenden Merkmal:
a. die Steuereinheit (10) ist zur Steuerung eines transendoskopischen Instruments (120) vorgesehen, welches durch einen Arbeitskanal eines Endoskops in den Körper eines Patienten ragt,
**gekennzeichnet durch** die folgenden zusätzlichen Merkmale:
b. die Steuereinheit (10) weist mindestens ein Steuerelement (20) auf, insbesondere in Form mindestens eines 2-Wege-Controllers (20) oder 4-Wege-Controllers (20) und/oder mindestens eines Betätigungsknopfes, und
c. die Steuereinheit (10) ist zur Anbringung an der Außenseite eines Endoskopschlauchs (110) ausgebildet und verfügt über einen Aufnahmebereich (14) zur Aufnahme des Endoskopschlauchs (110) sowie einen mit dem Endoskopschlauch (110) zusammenwirkenden Befestigungsmechanismus (50) und/oder über einen mit dem Endoskopschlauch (110) zusammenwirkenden Führungsmechanismus (52), oder
die Steuereinheit (10) ist zur Anlage an der Außenseite des Endoskopschlauchs (110) ausgebildet und verfügt über einen Anlagebereich (16) zur definierten Anlage am Endoskopschlauch (110).

2. Steuereinheit (10) nach Anspruch 1 mit dem folgenden weiteren Merkmal:
a. der Befestigungsmechanismus (50) ist derart ausgestaltet und/oder derart an den Endoskopschlauch angepasst, dass
- eine Schubkraft (2) in Erstreckungsrichtung des Endoskopschlauchs von mindestens 2 N, insbesondere vom mindestens 4 N, über den Befestigungsmechanismus (50) von der Steuereinheit (10) auf den Endoskopschlauch (110) übertragbar ist, und/oder
- ein Torsionsmoment (4) von mindestens 2 Nm, insbesondere von mindestens 4 Nm, über den der Befestigungsmechanismus (50) von der Steuereinheit (10) auf den Endoskopschlauchs (110) übertragbar ist.

3. Steuereinheit (10) nach Anspruch 1 oder 2 mit dem folgenden weiteren Merkmal:
a. der Befestigungsmechanismus (50) weist mindestens eine erste Haltefläche (52) und mindestens eine gegenüberliegende zweite Haltefläche (54) zur beidseitigen Anlage am Endoskopschlauch (110) auf, über die gemeinsam eine klemmende Verbindung mit dem Endoskopschlauch herstellbar ist, vorzugsweise mit dem folgenden zusätzlichen Merkmal:
b. der Befestigungsmechanismus (50) weist beidseitig des Endoskopschlauchs (110) jeweils mindestens zwei Halteflächen (52, 54) auf, die bezogen auf eine Erstreckungsrichtung des Endoskopschlauchs (110) voneinander beabstandet eine klemmende Verbindung mit dem Endoskopschlauch (110) gestatten.

4. Steuereinheit (10) nach einem der vorstehenden Ansprüche mit den folgenden weiteren Merkmalen:
a. der Befestigungsmechanismus (50) umfasst eine mittels einer Federeinrichtung (72C) gegen den Endoskopschlauch (110) gedrückte Haltefläche (54), und
b. die Steuereinheit (10) weist eine Betätigungsfläche (70B) zur manuellen Betätigung auf, mittels derer eine Haltefläche (54) gegen eine Rückstellkraft der Federeinrichtung (72C) vom Endoskopschlauch (110) weggedrückt werden kann.

5. Steuereinheit nach einem der vorstehenden Ansprüche mit dem folgenden weiteren Merkmal:
a. die Steuereinheit (10) verfügt über eine Haupteinheit (70), an der mindestens ein Steuerelement (20) vorgesehen ist, und über einen demgegenüber beweglichen, insbesondere schwenkbeweglichen Befestigungsträger (72), wobei auf Seite der Haupteinheit (70) und auf Seite des Befestigungsträgers (72) jeweils mindestens eine Haltefläche (52, 54) vorgesehen ist,
vorzugsweise mit mindestens einem der folgenden zusätzlichen Merkmale:
b. die Haupteinheit (70) und der Befestigungsträger (72) verfügen über zusammenwirkende Rasteinrichtungen (74, 76), durch die beim Festklemmen der Steuereinheit (10) am Endoskopschlauch (110) die Haupteinheit (70) und der Befestigungsträger (72) miteinander verrastet sind, wobei insbesondere eine Mehrzahl von Rastpositionen durch die Rasteinrichtungen (74, 76) definiert werden, und/oder
c. der Befestigungsträger (72) ist um eine Schwenkachse (6) gegenüber der Haupteinheit (70) verschwenkbar, wobei die Schwenkachse (6) im Wesentlichen orthogonal zur Aufnahme für den Endoskopschlauch ausgerichtet ist.

6. Steuereinheit (10) nach einem der vorstehenden Ansprüche mit dem folgenden weiteren Merkmal:
a. die Steuereinheit (10) weist eine ergonomische Formgebung auf, die spezifisch zur Führung der Steuereinheit (10) mit der rechten Hand oder der linken Hand ausgebildet ist,
vorzugsweise mit mindestens einem der folgenden zusätzlichen Merkmale:
b. die Steuereinheit (10) weist eine ergonomische Formgebung auf, die spezifisch zur Führung der Steuereinheit (10) mit der rechten Hand ausgebildet ist, und/oder
c. die Steuereinheit (10) weist mindestens einen 4-Wege-Controller (20) oder einen 2-Wege-Controller auf (20), der bei bestimmungsgemäßem Fassen der Steuereinheit (10) mit der Hand im Bereich des Daumens angeordnet ist.

7. Steuereinheit (10) nach einem der vorstehenden Ansprüche mit mindestens einem der folgenden weiteren Merkmale:
a. die Steuereinheit (10) weist eine seitliche Ausnehmung (56) auf, in die der Endoskopschlauch (110) eingeschoben werden kann, so dass er an einem vorderen Ende und an einem hinteren Ende aus der Steuereinheit (10) hinausragt, und/oder
b. die Steuereinheit (10) weist eine Haltehilfe in Form eines Fingerlochs (98) auf, in das der Bediener beim Greifen der Steuereinheit einen Finger einführt.

8. Steuereinheit (10) nach einem der vorstehenden Ansprüche mit dem folgenden zusätzlichen Merkmal:
a. das Steuerelement (20) ist als 4-Wege-Controller mit einem in Richtung zweier Achsen beweglichem Steuerabschnitt (22) ausgebildet, wobei der Steuerabschnitt in einer NeutralStellung mit der Ausnehmung für den Endoskopschlauch einen Winkel größer 0° und kleiner 90° einschließt, insbesondere einen Winkel größer 20° und kleiner 70°.

9. Endoskopzubehör mit den folgenden Merkmalen:
a. das Endoskopzubehör umfasst ein transendoskopisches Instrument (120) zur Durchführung durch einen Arbeitskanal (112) eines Endoskops, wobei das transendoskopische Instrument (120) über mindestens einen elektromechanisch betätigbaren Aktor verfügt, und
b. das Endoskopzubehör umfasst eine Steuereinheit (10) nach einem der vorstehenden Ansprüche zur Steuerung des mindestens einen Aktors.

10. Endoskopzubehör nach Anspruch 9 mit dem folgenden zusätzlichen Merkmal:
a. das transendoskopische Instrument (120) verfügt über ein Werkzeug (122), welches mittels des mindestens einen elektromechanisch betätigbaren Aktors gegenüber einer stirnseitigen Öffnung (116) des Arbeitskanals (112) verlagerbar ist,
vorzugsweise mit einem der folgenden zusätzlichen Merkmale:
b. das Werkzeug ist als bewegliches Messer für die endoskopische Submukosadissektion ausgebildet, oder
c. das Werkzeug ist als Hämostasewerkzeug ausgebildet, oder
d. das Werkzeug ist als endoskopischer Clipapplikator ausgebildet, oder
e. das Werkzeug ist als Sonde ausgebildet, insbesondere als APC-Sonde oder als RFA-Sonde oder als EUS-Sonde, oder
f. das Werkzeug ist als Injektionsnadel ausgebildet, oder
g. das Werkzeug ist als Nahtwerkzeug ausgebildet, oder
h. das Werkzeug ist als Biopsiezangen ausgebildet, oder
i. das Werkzeug ist als Schlinge ausgebildet, oder
j. das Werkzeug ist als Fangnetz ausgebildet, oder
k. das Werkzeug ist als bewegliches Papillotom ausgebildet, oder
l. das Werkzeug ist als beweglicher Gallengangsendoskop ausgebildet, oder
m. das Werkzeug ist als endoskopische Schere ausgebildet, oder
n. das Werkzeug ist als Ballondilatator ausgebildet.

11. Endoskopzubehör nach Anspruch 9 oder 10 mit mindestens einem der folgenden weiteren Merkmale:
a. das transendoskopische Instrument (120) und die Steuereinheit (10) sind über eine drahtlose Übertragungstechnik miteinander verbunden, oder
b. das transendoskopische Instrument (120) und die Steuereinheit (10) sind über mindestens ein Steuerkabel miteinander verbunden.

12. Endoskopzubehör nach einem der Ansprüche 9 bis 11 mit dem folgenden weiteren Merkmal:
a. das transendoskopische Instrument (120) und die Steuereinheit (10) sind fest miteinander gekoppelt und durch den Verwender nicht mit anderen transendoskopischen Instrumenten oder Steuereinheiten koppelbar,
vorzugsweise mit mindestens einem der folgenden zusätzlichen Merkmale:
b. das transendoskopische Instrument (120) und die Steuereinheit (10) sind als Einweg-Komponenten ausgebildet, und/oder
c. das transendoskopische Instrument (120) weist eine Steuerelektronik auf, in der eine eindeutige Geräte-ID der Steuereinheit hinterlegt ist, wobei die Steuerelektronik dafür ausgebildet ist, eine Steuerung des Aktors nur zu ermöglichen, wenn darauf gerichtete Steuerbefehle von der Steuereinheit mit der hinterlegten Geräte-ID stammen.

13. Endoskopzubehör nach Anspruch 9 bis 12 mit dem folgenden weiteren Merkmal:
a. das transendoskopische Instrument (120) und die Steuereinheit (10) kommunizieren über einen kryptographisch geschützten Kanal.

14. Endoskopsystem (100) mit den folgenden Merkmalen:
a. das Endoskopsystem (100) verfügt über ein Endoskop mit einem flexiblen Endoskopschlauch (110), in dem ein Arbeitskanal (112) für ein transendoskopisches Instrument (120) vorgesehen ist, und
b. das Endoskopsystem (100) verfügt über eine Endoskopzubehör nach einem der Ansprüche 9 bis 13.

15. Endoskopsystem (100) nach Anspruch 14 mit dem folgenden zusätzlichen Merkmal:
a. die Steuereinheit (10) des Endoskopzubehörs ist derart an den flexiblen Endoskopschlauch (110) angepasst, dass über den Befestigungsmechanismus
- eine Schubkraft in Erstreckungsrichtung des Endoskopschlauchs (110) von mindestens 2 N, insbesondere vom mindestens 4 N, von der Steuereinheit (10) auf den Endoskopschlauch (110) übertragbar ist, und/oder
- ein Torsionsmoment von mindestens 2 Nm, insbesondere von mindestens 4 Nm, von der Steuereinheit (10) auf den Endoskopschlauch (110) übertragbar ist.
